# EUROPEAN PATENT APPLICATION

(11) **EP 3 893 245 A1**
(43) Date of publication of application: **13.10.2021**
(21) Application number: 20169179.7
(22) Date of filing: 10.04.2020
(51) Int. Cl.: G16H 30/40, G16H 40/63, G16H 50/70

(54) **CORRECTION OF AUTOMATED LABEL DATA FOR TOMOGRAPHIC MEDICAL IMAGING SYSTEMS**

(71) Applicant: Koninklijke Philips N.V., 5656 AG Eindhoven (NL)
(72) Inventor: SOMMER, Karsten, 5656 AE Eindhoven (NL); KRUEGER, Sascha, 5656 AE Eindhoven (NL); KOKEN, Peter, 5656 AE Eindhoven (NL); SENEGAS, Julien, 5656 AE Eindhoven (NL)
(74) Representative: Philips Intellectual Property & Standards

(57) **Abstract**

Disclosed herein is a medical imaging system (100) configured for acquiring tomographic medical imaging data (256) from a subject (118) within an imaging zone (108). The medical imaging system comprises a subject support (120) with a support surface (122). The medical imaging system further comprises a camera system (124) configured for acquiring optical image data (160) descriptive of the support surface and the subject when subject support is in a first position (128). The medical imaging system further comprises a computational system (130) configured for controlling the medical imaging system. The execution of machine executable instructions (150) causes the computational system to: control (300) the camera system to acquire the optical image data; receive (302) scan label data (162) by inputting the optical image data into an artificial intelligence system (152); render (304) the scan label data as an overlay (170) using a user interface (136); provide (306) a scan label data modifier control (168) using the user interface, wherein the scan label data modifier control is configured to receive modified scan label data (170); and construct (308) annotated training data (172) from the optical image data and the modified scan label data if the scan label data is received.

## Description

### FIELD OF THE INVENTION

The invention relates to tomographic medical imaging system, in particular to systems for automated configuration and positioning for tomographic medical imaging.

### BACKGROUND OF THE INVENTION

In tomographic medical imaging modalities such as Magnetic Resonance Imaging (MRI), Computed Tomography (CT), Positron Emission Tomography (PET), and Single Photon Emission Tomography (SPECT) the proper configuration of the subject as well as any fixtures or other medical equipment may be difficult. Various types of artificial intelligence may be used to assist an operator, however often times the operators do not use this assistance because of concerns on the accuracy these artificial intelligence systems.

European patent publication EP 2 486 847 A1 discloses a system for identifying an area of interest on a surgical image, including a source of surgical image data, which may be a camera, an image processing unit, which may be a camera control unit, and a destination, which may be a display. The image processing unit is configured to receive the surgical image data and combine it with an overlay pattern for identifying an area of interest, which is then displayed on the display. The overlay may include a key with coordinates or labels. Properties of the overlay and the key may be customized and adjusted.

### SUMMARY OF THE INVENTION

The invention provides for a medical imaging system, a method of operating the medical imaging system and a computer program comprising machine-executable instructi ons.

Embodiments of the invention may provide for an improved method of ensuring quality during the acquisition of tomographic images. Embodiments may provide this by using an optical imaging system to acquire optical imaging data that is input into an artificial intelligence system that outputs scan label data. The scan label data is then provided as an overlay which the user can modify. If the user decides to modify the overlay annotated training data can be generated directly that may be used to improve the functioning of the artificial intelligence system.

In some examples the annotated training data may be used immediately to train the artificial intelligence system. After training the data may be deleted. This immediate training and deletion of the data may provide for improved data security. If the annotated training data has been deleted it is impossible for it to be stolen by a hacker. It also may help to eliminate the technical need to make the annotated training data anonymous to protect the privacy of the subject being imaged.

In one aspect the invention provides for a medical imaging system that is configured for acquiring tomographic medical imaging data from a subject within an imaging zone. The medical imaging system comprises a subject support with a support surface. The support surface is configured to receive and support a subject. For example, the subject may lie or repose on the support surface. The subject support is configured for transporting the subject from a first position to a second position. In the second position the subject is at least partially within the imaging zone.

The medical imaging system further comprises a camera system configured for acquiring optical image data descriptive of the support surface and the subject when the subject support is in the first position. The camera system and optical image data may be in the visible wave length as well as in the infra-red wave length range.

The medical imaging system further comprises a user interface. The medical imaging system further comprises a memory storing machine-executable instructions and an artificial intelligence system. The artificial intelligence system is configured to output scan label data in response to receiving the optical image data as input. The output scan label data may for example take different forms in different examples. This may include anatomical landmarks of the subject, a coil position in the case of magnetic resonance imaging, and a subject outline as an example. For example, the output scan label data may also be referred to as output scan landmark data or anatomical landmark data in some examples.

The medical imaging system further comprises a computational system configured for controlling the medical imaging system. Execution of the machine-executable instructions further causes the computational system to control the camera system to acquire the optical image data with the subject support in the first position. Execution of the machine-executable instructions further causes the computational system to receive the scan label data by inputting the optical image data into the artificial intelligence system. Execution of the machine-executable instructions further causes the computational system to render the scan position data as an overlay using the user interface. The overlay may take different forms in different examples also. For example, the overlay may be a display or rendering on a display over other images. In other examples the overlay may be a natural projection onto the subj ect.

Execution of the machine-executable instructions further causes the computational system to provide a scan label data modifier control using the user interface. The scan label data modifier control is configured to receive modified scan label data. For example, if the operator of the medical imaging system identifies the overlaid scan label data as incorrect or inaccurate the operator can use the scan label data modifier to produce the modified scan label data. Execution of the machine-executable instructions further causes the computational system to construct annotated training data from the optical image data and the modified scan label data if the scan label data is received. This embodiment may be beneficial because it may provide an efficient means for the operator of the medical imaging system to produce annotated training data which may be used to train or modify the artificial intelligence system.

The artificial intelligence system may take different forms in different examples. In some examples the artificial intelligence system comprises a neural network or convolutional neural network. In either case the neural network can be trained by providing it with the annotated training data. For example, the medical imaging system can have its artificial intelligence system trained during use. In other examples the neural network of the artificial intelligence system can be trained using pre-supplied annotated data.

The output scan label data as mentioned above may take different forms. In some examples they are anatomical landmarks which are overlaid either on a display or projected on the subject. This may for example identify the location of particular portions of the subject or joint locations. It may also indicate drive position of various organs or other anatomical structures. The output scan label data may in other examples be for support surfaces or other objects such as magnetic resonance imaging coils, headphones, tubes, cables, contrast agent injectors, etc. In another example it may be very useful to provide an outline of the subject to estimate the subject's position, weight, or if the subject may collide with a portion of the medical imaging system when the subject is moved from the first position to the second position. The operator can use the scan label data modifier control to modify the outline and correct the efficiency of the system.

In another embodiment execution of the machine-executable instructions further causes the processor to train the artificial intelligence system using the annotated training data immediately. So as the annotated training data is constructed the artificial intelligence system is trained right away. For example, if the artificial intelligence system comprises a neural network then the neural network may for example be trained using a deep learning algorithm. Execution of the machine-executable instructions further causes the processor to delete the annotated training data after training. This may have the advantage that the subject's confidential data is not retrained but is still used for improving the artificial intelligence system. This may for example contribute to the data security of the subject. This may also reduce the need to store or retain this data.

In another embodiment execution of the machine-executable instructions further causes the processor to log the annotated training data. In this example instead of training and then deleting the data immediately it is stored for later training. This may for example have the advantage of being able to collect the data from multiple sites.

In another embodiment the artificial intelligence system comprises a convolutional neural network and a scan position data generator. The convolutional neural network is configured for outputting the scan label data in response to receiving the optical image data. The scan position data generator is configured for generating the scan position data in response to receiving the scan label data. The scan position data may for example provide data about the overall position and positioning of the subject. For example, the artificial intelligence system may comprise the convolutional neural network for providing the scan label data and then use a second artificial intelligence module to provide the scan position data generator. This may have the advantage of making the output of the system more flexible and robust. This for example may be because the scan label data is easier to understand and therefore easier to correct for the operators. The scan position generator can also be implemented using a learning algorithm such as a linear regression that is easier for operators to understand and/or correct.

In another embodiment the scan position data generator comprises a rule-based system. In this example the operator of the medical imaging system may program or provide rules to construct the scan position data generator. This may require more manual work but it may for example be more transparent.

In another embodiment the scan position data generator comprises a trained neural network.

In another embodiment the scan position data generator comprises a decision tree.

In another embodiment the scan position data generator comprises a support vector machine.

In another embodiment the scan position data generator comprises a principle component analysis algorithm.

In another embodiment the scan position data generator comprises a combination of one or more of the above mentioned.

In another embodiment the user interface comprises a projection system configured for projecting the overlay on the subject. For example, there may be a projector which projects the determined outline, anatomical markers, the location of various joints and other items directly on the subject.

In another embodiment the user interface comprises a screen configured for displaying the optical image data and the overlay on the optical image data. This may be beneficial because the operator of the medical imaging system can directly see how effective the artificial intelligence system is in producing the output scan label data.

In another embodiment the scan label data modifier control is implemented using a gesture recognition system. For example, the same camera system that is used to obtain the optical image data may be used to register the gestures of an operator also in its optical field.

In another embodiment the scan label data modifier control comprises a touchscreen. For example, the touchscreen may implement a graphical user interface or an implementation of the scan label data modifier control.

In another embodiment the scan label data modifier control is implemented using a mouse.

In another embodiment the scan label data modifier control is implemented using a graphical user interface. This for example may be implemented on a touchscreen or on a conventional computer monitor or display.

In another embodiment the scan label data comprises a subject pose.

In another embodiment the scan label data comprises a subject position.

In another embodiment the scan label data comprises a subject outline.

In another embodiment the scan label data comprises a subject weight estimate.

In another embodiment the scan label data comprises a subject skeletal overlay. This for example may include the location of joints of the subject. The determination of the subject's skeletal overlay may be particularly beneficial in determining the location of other organs as well as the subject pose or position.

In another embodiment the scan label data comprises an organ identification. For example, it may identify the location and position of various organs.

In another embodiment the scan label data comprises a location of a region of interest. For example, if a region is to be imaged with a tomographic imaging system such as an MRI or CT system the region of interest is the area from which the data is acquired.

In another embodiment the scan label data comprises a cable. For example, there may be various cables or other fixtures and these may be identified by the scan label data.

In another embodiment the scan label data comprises an antenna location. For magnetic resonance imaging there may be various surface coils or antennas which are placed on or adjacent to the subject. A knowledge of the antenna location with respect to the position of the subject may be useful in determining if the system is configured properly.

In another embodiment the scan label data comprises a support location. The support location may for example be the location of a pillow or other support or rest for the subj ect.

In another embodiment the scan label data comprises a subject outline. This may be an outline of the subject so that an estimate of the subject's position may be determined.

In another embodiment the scan label data comprises a tube location. For example, there may be various tubes to provide or retrieve fluids from the subject.

In another embodiment the scan label data comprises a contrast agent injector location. This may be useful in determining if the contrast agent injector will interfere with the proper imaging or operation of the medical imaging system.

In another embodiment the scan label data comprises medical equipment location. There may be various medical equipment or fixtures and a knowledge of their location may be useful in properly configuring the medical imaging system.

In another embodiment execution of the machine-executable instructions further causes the processor to receive scan parameters. The artificial intelligence system is configured to output the scan label data in response to receiving the optical image data and the scan parameters as input. The scan parameters may for example be data which is descriptive of the conditions under which the medical imaging system will acquire the tomographic medical imaging data. These may be used to further configure the preferences of the operator or the location operating the medical imaging system.

In another embodiment the scan parameters comprise an operator identifier. This may for example be a particular operator or physician operating the medical imaging system. This may be useful for example in setting the preferences of the medical imaging system for a particular operator.

In another embodiment the scan parameters further comprise an ordering physician identifier. This may for example be useful in training the medical imaging system to provide the output scan label data in a means which fits for a particular physician's preferences.

In another embodiment the scan parameters comprise a location identifier. This may for example be the identification of a particular clinic or physical location. This may be useful in customizing the operation of the artificial intelligence system for a particular clinic.

In another embodiment the scan parameters comprise a medical imaging protocol identifier. The medical imaging protocol identifier may for example identify a particular protocol used to operate and acquire medical imaging data from the subject. For example, there may be different types of magnetic resonance imaging protocols and for different types there may be different preferences as to how the output scan label data is produced.

In another embodiment the scan parameters comprise a magnetic resonance imaging antenna identifier. For example, different types of magnetic resonance imaging antennas may be placed on the subject. A knowledge in advance of the particular antennas used may help the output scan label data to be produced more accurately, and to identify incorrect selection of antennas by the operator.

In another embodiment the medical imaging system is a magnetic resonance imaging system.

In another embodiment the medical imaging system is a computed tomography system.

In another embodiment the medical imaging system is a positron emission tomography system.

In another embodiment the medical imaging system is a single photon emission tomography system.

In another embodiment the memory contains medical imaging system control commands configured to control the medical imaging system to acquire the tomographic medical imaging data according to a medical imaging protocol. Execution of the machine-executable instructions further causes the processor to modify the medical imaging control commands using the scan label data. For example, the scan label data may provide detailed information about the relationship and the subject pose, subject position, as well as their relation to other supports or other medical equipment. Execution of the machine-executable instructions further causes the processor to control the medical imaging system with the medical imaging system control commands to acquire the tomographic medical image data.

In another aspect the invention provides for a method of operating a medical imaging system configured for acquiring tomographic medical imaging data from a subject within an imaging zone. The medical imaging system comprises a subject support with a support surface. The support surface is configured to receive and support a subject. The subject support is configured for transporting the subject from a first position to a second position. In the second position the subject is at least partially within the imaging zone. The medical imaging system further comprises a camera system configured for acquiring optical image data descriptive of the support surface and the subject when the subject support is in the first position.

The medical imaging system further comprises a user interface. The medical imaging system further comprises a memory storing an artificial intelligence system. The artificial intelligence system is configured to output scan label data in response to receiving the optical image data as input. The method comprises controlling the camera system to acquire the optical image data. The method further comprises receiving the scan label data by inputting the optical image data into the artificial intelligence system. The method further comprises rendering the scan label data as an overlay using the user interface. The method further comprises providing a scan label data modifier control using the user interface. The scan label data modifier control is configured to receive modified scan label data. The method further comprises constructing annotated training data from the optical image data and the modified scan label data if the scan label data is received.

In another aspect the invention provides for a computer program comprising machine-executable instructions for execution by a computational system controlling a medical imaging system. For example, the computer program may be provided in the form of a computer program product. The medical imaging system is configured for acquiring tomographic medical imaging data from a subject within an imaging zone. The medical imaging system comprises a subject support with a support surface. The support surface is configured to receive and support a subject.

The subject support is configured for transporting the subject from a first position to a second position. In the second position the subject is at least partially within in the imaging zone. The medical imaging system further comprises a camera system configured for acquiring optical image data descriptive of the support surface and the subject when the subject support is in the first position. The medical imaging system further comprises a user interface. The medical imaging system further comprises a memory storing machine-executable instructions and an artificial intelligence system. The artificial intelligence system is configured to output scan label data in response to receiving the optical image data as input.

Execution of the machine-executable instructions causes the computational system to control the camera system to acquire the optical image data. Execution of the machine-executable instructions further causes the computational system to receive the scan label data by inputting the optical image data into the artificial intelligence system. Execution of the machine-executable instructions further causes the computational system to render the scan label data as an overlay using the user interface.

Execution of the machine-executable instructions further causes the computational system to provide a scan label data modifier control using the user interface. The scan label data modifier control is configured to receive modified scan label data. Execution of the machine-executable instructions further causes the processor to construct annotated training data from the optical image data and the modified scan label data if the scan label data is received.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a processor" should be interpreted as possibly containing more than one processor or processing core. The processor may for instance be a multi-core processor. A processor may also refer to a collection of processors within a single computer system or distributed amongst multiple computer systems. The term computational system should also be interpreted to possibly refer to a collection or network of computing devices each comprising a processor or processors. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high level language or in a pre-compiled form and be used in conjunction with an interpreter which generates the machine executable instructions on the fly. In other instances the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a magnetic resonance imaging system;
Fig. 2 shows a further view of the magnetic resonance imaging system of Fig. 1;
Fig. 3 shows a flow chart which illustrates a method of operating the magnetic resonance imaging system of Figs. 1 and 2;
Fig. 4 illustrates an example of optical image data with a rendering of scan label data as an overlay; and
Fig. 5 illustrates a further example of optical image data with a rendering of scan label data as an overlay.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

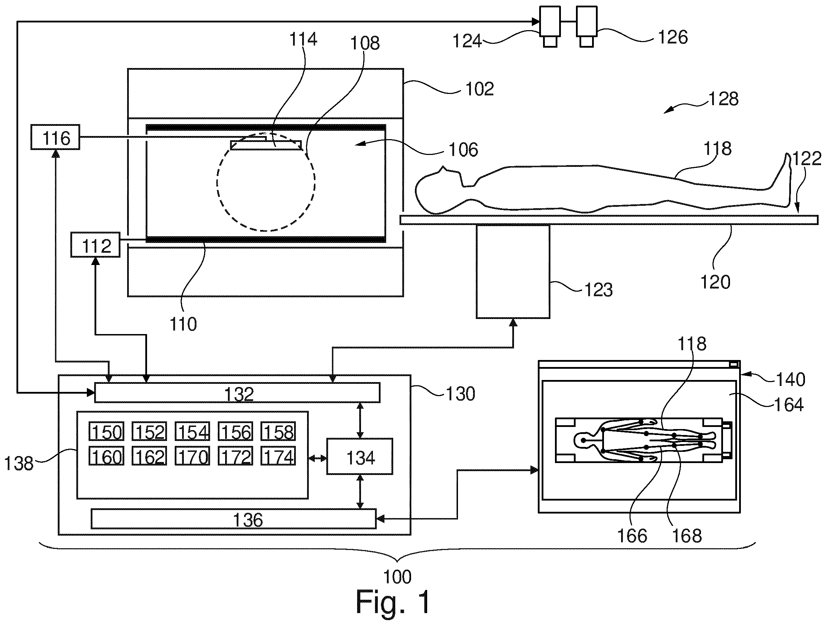

Fig. 1 illustrates an example of a magnetic resonance imaging system 100. The magnetic resonance imaging system 100 is intended to represent a medical imaging system according to an example. The features of the magnetic resonance imaging system 100 may for example be replaced by a computed tomography system, a positron emission tomography system, a single photon emission tomography system or other tomographic imaging system.

The magnetic resonance imaging system comprises a magnet 102. The magnet 102 may for example be a superconducting magnet. Alternatively, the magnet 102 may be a resistive type magnet.

The use of different types of magnets is also possible; for instance it is possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 106 of the cylindrical magnet 102 there is an imaging zone 108 where the magnetic field is strong and uniform enough to perform magnetic resonance imaging.

Within the bore 106 of the magnet there is also a set of magnetic field gradient coils 110 which is used for acquisition of preliminary magnetic resonance data to spatially encode magnetic spins within the imaging zone 108 of the magnet 102. The magnetic field gradient coils 110 connected to a magnetic field gradient coil power supply 112. The magnetic field gradient coils 110 are intended to be representative. Typically magnetic field gradient coils 110 contain three separate sets of coils for spatially encoding in three orthogonal spatial directions. A magnetic field gradient power supply supplies current to the magnetic field gradient coils. The current supplied to the magnetic field gradient coils 110 is controlled as a function of time and may be ramped or pulsed.

Adjacent to the imaging zone 108 is a radio-frequency coil 114 for receiving radio transmissions from spins also within the imaging zone 108. In some examples, the radio-frequency coil may also be configured for manipulating the orientations of magnetic spins within the imaging zone 108. The radio frequency antenna may contain multiple coil elements. The radio frequency antenna may also be referred to as a channel or antenna. The radio-frequency coil 114 is connected to a radio frequency reciever or transceiver 116. The radio-frequency coil 114 and radio frequency transceiver 116 may be optionally replaced by separate transmit and receive coils and a separate transmitter and receiver. It is understood that the radio-frequency coil 114 and the radio frequency transceiver 116 are representative. The radio-frequency coil 114 could also represent a dedicated transmit antenna and a dedicated receive antenna. Likewise the transceiver 116 may also represent a separate transmitter and receivers. The radio-frequency coil 114 may also have multiple receive/transmit elements and the radio frequency transceiver 116 may have multiple receive/transmit channels. For example if a parallel imaging technique such as SENSE is performed, the radio-frequency could 114 will have multiple coil elements.

The subject support 120 has a support surface 122 configured for receiving and supporting the subject 118. The subject support 120 is further connected to an actuator 123. In this image the subject support 120 is shown in a first position 128. In the first position 128 there is a camera 124 that is configured for acquiring optical image data 160. There is also an optional projector 126 which may be configured for overlaying a copy of the scan label data on the subject 118 directly.

The magnetic resonance imaging system comprises computational system 130 is configured for controlling the operation and function of the magnetic resonance imaging system 100. The computational system 130 comprises a hardware interface that is connected to the magnetic field gradient coil power supply, the transceiver 116, the camera system 124 and the optional projector 126. The computational system 130 further comprises a processor 134 and a user interface 136. The user interface 136 in this example is shown as comprising a graphical user interface 140. The graphical user interface 140 may be different in different examples.

In some instances, it may be rendered on a screen and a mouse may be used to provide or edit data. In other examples the graphical user interface 140 may be provided on a touchscreen. In yet other examples the graphical user interface 140 is provided using the optional projector system 126. In this case other conventional means of editing such as a mouse or touchscreen may be used in addition. In some instances, the camera system 124 may also image an operator of the magnetic resonance imaging system 100 such that various gestures may be used to edit overlaid scan label data.

The processor 134 is also shown as being connected to a memory 138. The memory 138 is intended to represent various types of memory which may be accessible to the processor 134. The memory 138 may for example include a non-transitory storage medium.

The memory 138 is shown as containing machine-executable instructions. The machine-executable instructions 150 enable the computational system 130 to perform various computational and data processing tasks. The machine-executable instructions 150 may also enable the computational system 130 to control the operation and function of the medical imaging system or magnetic resonance imaging system in this case 100. The memory 138 is further shown as containing an artificial intelligence system 152.

The artificial intelligence system 152 is configured to output scan label data in response to receiving the optical image data as an input. The artificial intelligence system 152 may be implemented in different ways. In this example the artificial intelligence system 152 comprises a neural network 154 and an optional scan position data generator 156. The memory 138 is further shown as containing pulse sequence commands 158. The pulse sequence commands are commands or data which may be converted into such commands which enable the computational system 130 to control the magnetic resonance imaging system 100 to acquire k-space data. The memory 138 is further shown as containing optical image data 160 that was acquired when the subject support 120 was in the first position 128. The memory 138 is further shown as containing scan label data 162 that is received by inputting the optical image data 160 into the artificial intelligence system 152. In this case the neural network 154 output this data.

On the graphical user interface 140 there is a rendering of the optical image data 164 and an overlay 168 of the scan label data 166. In this case the scan label data 162 is an indication of joint locations of the subject 118. The graphical user interface is constructed such that there is a scan label data modifier control 168. In this case the operator can choose the locations of the joints and move them. In different situations the data may be differently arranged. For example, if the scan label data 162 comprises such things as a patient outline the graphical user interface 140 may enable the user to modify or correct the outline of the subject 118.

The memory 138 is further shown as containing modified scan label data 170 that was received from the graphical user interface 140. The memory 138 is further shown as containing annotated training data 172 that was constructed from the modified scan label data 170 and the optical image data 160. The memory 174 is further shown as containing a training module 174 which may be used to train the neural network 154 using the annotated training data 172 and the optical image data 160. For example, in this clinical situation as the system is used the artificial intelligence 152 is routinely improved. Various parameters may be additionally passed to the artificial intelligence system 152. For example, a particular location of the magnetic resonance imaging system 100 as well as particular operators or physicians ordering routines. An advantage of having the training module 174 in the memory 138 is that after the artificial intelligence system 152 and in particular the neural network 154 has been trained the optical image data 160, as well as the scan label data 162, modified scan label data 170 and annotated training data 172 may be deleted. This may provide for improved security of the subject's 118 personal data.

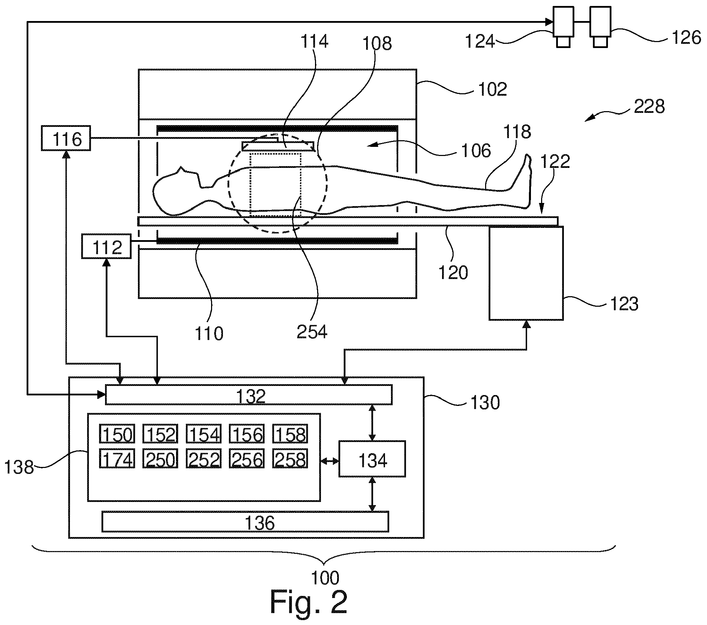

Fig. 2 shows the subject support 120 in the second position 228. In this position the subject support 120 has moved the subject 118 such that the subject 118 is at least partially within the imaging zone 108.

The memory 138 is further shown as containing optional scan position data 250 that was produced by inputting the scan label data 162 or the modified scan label data 170 into the scan position data generator 156. The optional scan position data 250 can for example be used by the machine-executable instructions to modify the pulse sequence commands 158. The memory is shown as containing modified pulse sequence commands 252. The modified pulse sequence commands 252 may for example specify a region of interest 254 from where to acquire k-space data. The memory 138 is shown as containing k-space data 256 that was acquired using either the modified pulse sequence commands 252 or the pulse sequence commands 158. The memory is further shown as containing a magnetic resonance image 258 that was reconstructed from the k-space data 256.

One feature of note in Fig. 2 is that the optical image data 160, the scan label data 162, the modified scan label data 170, and the annotated training data 172 have been deleted from the memory 138. This for example protects the privacy and data security of the subject 118. Also, by deleting the data, it completely eliminates the possibility of it being stolen by a hacker for example. In other examples this data could for example be logged in a database and used for training systems in a distributed manner.

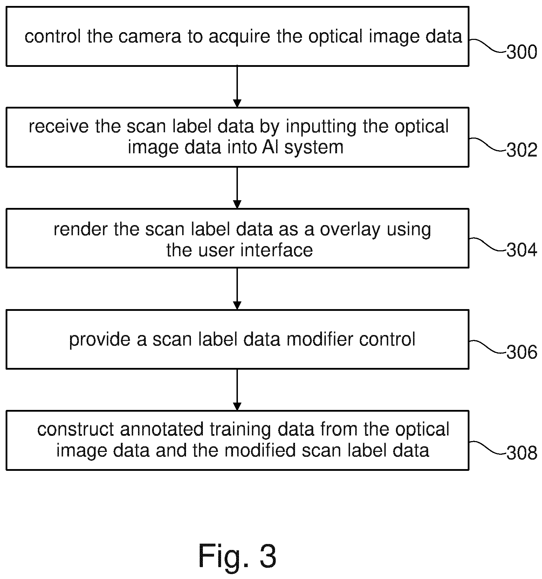

Fig. 3 illustrates one method of operating the magnetic resonance imaging system 100 of Fig. 1 and 2. First in step 300 the camera system 124 is controlled to acquire the optical image data 160. Next in step 302 the scan label data 162 is received by inputting the optical image data 160 into the artificial intelligence system 152. Next in step 304 the scan label data 162 is rendered 164 as an overlay using the user interface 136. This for example could be done using the projection system 126 or the graphical user interface 140. Next, in step 306, the scan label data modifier control 168 is provided. In different examples this may be implemented differently. In one example there may be a graphical user interface 140 as is shown in Fig. 1 where various points 168 may be modified directly by a mouse or touchscreen. In other examples the overlay may be provided by the projector 126 and the camera system 124 may be used to interpret gestures from optical images which are then transformed into gestures for modifying the scan label data 162. Finally, in step 308, the annotated training data 172 is constructed from the optical image data 160 and the modified scan label data 170.

Examples may provide a means to provide visual feedback (via an overlay 166) to the operator for semi- or fully automated MRI exams (or other tomographic imaging modalities) based on external camera devices (camera 124) is described. The system may provide direct insight into the underlying mechanisms for selection of automatically selected exam parameters such as scan position or coil location. This feedback facilitates detection of erroneous behavior, e.g. due to incorrectly detected anatomical landmarks. For such cases, a method for quick correction of these inaccuracies is described. The system therefore enables automatic collection of valuable annotated data (annotated training data 172) in the field, exactly for those cases where system performance is inadequate. The resulting annotated dataset may therefore be used to improve the system's performance for rare failure cases.

Preparation of the subject for an MR exam (or other tomographic imaging scan) is a time-consuming and error-prone task. To simplify and accelerate the clinical workflow, automation of this part of the MR exam using external cameras may be used. Acceptance of such systems for automation by the clinical staff, however, may be hampered if the system's behavior appears inexplicable or if detection of system failure is difficult.

Moreover, any automated system for patient preparation will show incorrect behavior in certain extreme cases. This is particularly relevant for systems based on neural networks, where any input that is substantially different from the training dataset may result in unpredictable system behavior. Successful adoption of such automated systems in the field requires a straightforward user option to override the system's output. In these cases, retrospective improvement of the system such that it provides the correct user-defined output for the given situation would be highly desirable to continuously improve the system after deployment. Against this need, the difficulty to collect data from clinical sites for storage and processing within the company represents a major challenge.

Examples may provide a solution which overcomes this by allowing to retrain directly on-site. So, not only removes this the need for transfer of the data outside the hospital, it also removes the need for permanent storage.

Examples may provide intuitive visual feedback to the user for automated MR exams based on external camera devices. The visual feedback consists of an image that is acquired by the external camera (Monochrome, RGB, Infrared, Depth, ...) and an overlay (166) that visualizes image features that were detected by the underlying automation system (artificial intelligence system 152).

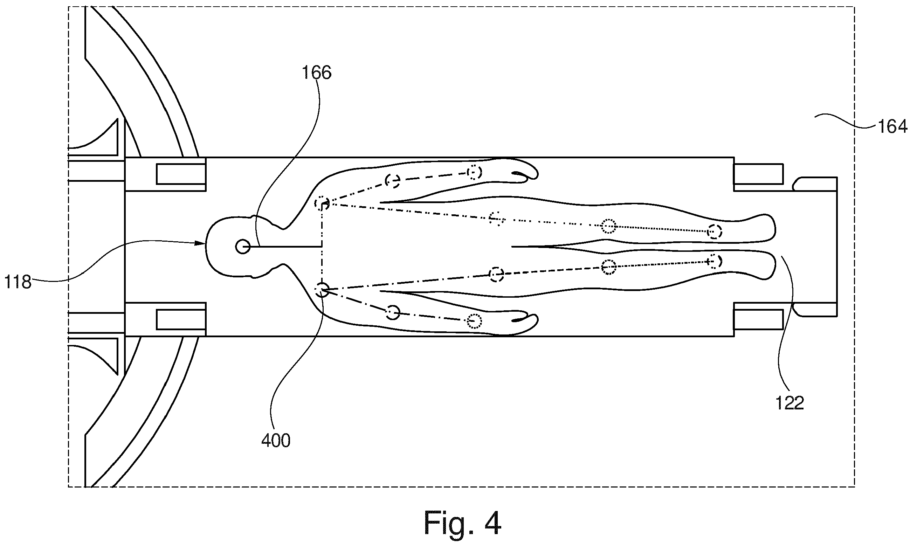

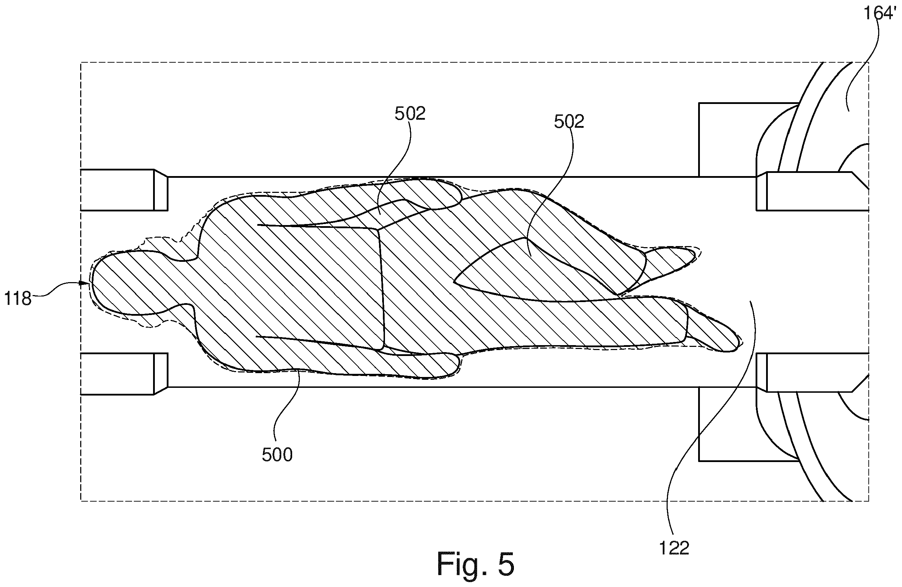

Depending on the targeted tasks for automation, one or more of the following different image features may be displayed:
- For automated selection of the scan position, anatomical landmarks such as the head, shoulders, elbows, wrists/hands, hips, knees, or ankles/feet are visualized using e.g. a simplified skeleton overlay (cf. Fig. 4 below).
- For automated detection and localization of RF coils, the coil position and spatial extent are visualized by a transparent overlay. In case of multiple coils, different colors may be used.
- To estimate the patient's height and weight as well as to avoid certain safety risks such as finger pinching or collision between patient and scanner bore, the patient outline can be detected automatically. This image feature is displayed as an overlay to the camera image using a transparent mask (cf. Fig. 5 below).
- Safety-related objects such as cables, tubes, contrast agent injectors, or other medical equipment are displayed as a (possibly transparent) overlay.

The visual feedback may be displayed on a video screen that is located directly on the scanner, i.e. at the scanner's gantry, or in a different location such as the scanner's control room. In all cases, visualization may be realized as a real-time or slightly delayed video stream, where both the camera output as well as the image feature visualization are updated with a high frame rate.

The example visualization methods may enable quick detection of system failure, such as incorrect localization of anatomical landmarks. In such cases, the operator is given two choices:
1) Immediate correction of the extracted image features by the operator: at least one of the video screens that display the visual feedback are sensitive to touch (touch screen) and include a software with correction capability. A drag-and-drop gesture is used to shift the landmark to the anatomically correct location. A similar method is used to correct placements of RF coils or other objects (see above). In case of inaccuracies in the patient outline determination, corrections can be realized using simple mask modifications, i.e. tools to erase or extend parts of the outline.
2) The operator may in some examples also override the system output by continuing with a "manual" patient preparation, i.e. using manual selection of the scan position, ignoring warnings due to misplaced coils or potential collisions, etc. The system will automatically annotate the acquired camera images and calculated image features as incorrect. To also obtain corrected annotations in this case, the image features are shown to the operator after completion of the patient preparation in the control room, i.e. during execution of the MR scan. This can be beneficial because many MR exams include relatively long sequences during which no user interaction is required. In this scenario, common peripheral devices (mouse, keyboard) are used instead of a touch screen. In particular, the MR console computer may be used.

In any case, the camera images(s) (optical image data 160) along with the corrected annotations (annotated training data) may be stored to be used for improvement of the system's performance. In case of a system based on neural networks, this annotated data is used to re-train the network, thereby reducing the system's error rate.

Figs. 4 and 5 illustrate examples of optical image data that is overlaid with label data. Both are examples of visual feedback for camera-based automated MR exams. Fig. 4 shows a skeletal or joint position overlay on an Infrared image for visualization of detected anatomical landmarks. Fig. 5 shows a transparent mask overlay on Monochrome image for visualization of detected body outline.

Fig. 4 shows an example of optical image data 164 that has the overlay of the scan label data 166. In this example the overlay 166 is a subject skeletal overlay. In this the position of joints and connection between these joints is displayed. This for example may be very useful in determining the pose of a subject and/or also determining the proper placement of a region of interest 254. On a touchscreen or graphical user interface the operator could very easily drag or move the position of individual joints 400.

Fig. 5 shows a further rendering of optical image data 164'. Again, a subject 118 is reposing on the support surface 122. In this case the scan label data is an outline 500 of the subject 118. This may be useful for example in identifying the weight and the possibility of a subject 118 colliding with a portion of the medical imaging system. In this example it can be seen that the outline 500 has several mistakes. There is an incorrect region between the legs and another incorrect region between the arm and trunk of the subject 118. The operator could for example go in and edit the outline 500 using the scan label data modifier control.

Examples may provide for broader acceptance of MR exam automation (or automation of other tomographic imaging modalities) due to its understandable user feedback. In addition, it may also include an override-feature to enable a correct exam preparation even in case of incorrect predictions by the system. This feature also allows to possibly store the user-corrected image features (annotated training data) along with the corresponding camera images to further improve the system after deployment. Both aspects ensure that the human operator remains the capable and fundamental control point of the technology.

In one example, several different system outputs with similar probability are shown to the user, e.g. a range of skeletons that are relatively consistent with the current camera image. The user can then simply select the correct configuration.

In another example, a projector 126 that is, for example, mounted on the ceiling of the MR room is used to visualize the image features directly onto the support surface 122 of the subject support 120. In this scenario, correction of inaccurate system output can be carried out using pre-defined gestures or tailored markers, such as patches with high infrared reflectivity, in combination with voice control.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: magnetic resonance imaging system
- 102: magnet
- 106: bore of magnet
- 108: imaging zone
- 110: magnetic field gradient coils
- 112: magnetic field gradient coil power supply
- 114: radio-frequency coil
- 116: transceiver
- 118: subject
- 120: subject support
- 122: support surface
- 123: actuator
- 124: camera system
- 126: optional projector
- 128: first position
- 130: computational system
- 132: hardware interface
- 134: processor
- 136: user interface
- 138: computer memory
- 140: graphical user interface
- 150: machine executable instructions
- 152: aritifical intelegence system
- 154: neural network
- 156: scan position data generator
- 158: pulse sequence commands
- 160: optical image data
- 162: scan label data
- 164: rendering of optical image data
- 164': rendering of optical image data
- 166: overlay of scan label data
- 168: scan label data modifier control
- 170: modified scan label data
- 172: annotated training data
- 174: training module
- 228: second position
- 250: optional scan position data
- 252: modified pulse sequence commands
- 254: region of interest
- 256: k-space data
- 258: magnetic resonance image
- 300: control the camera system to acquire the optical image data
- 302: receive the scan label data by inputting the optical image data into the artificial intelligence system
- 304: render the scan position data as an overlay using the user interface
- 306: provide a scan label data modifier control using the user interface
- 308: construct annotated training data from the scan label data and the modified scan label data if the scan label data is received
- 500: subject outline
- 502: incorrection region

## Claims

1. A medical imaging system (100) configured for acquiring tomographic medical imaging data (256) from a subject (118) within an imaging zone (108), wherein the medical imaging system comprises:
- a subject support (120) with a support surface (122), wherein the support surface is configured to receive and support the subject, wherein the subject support is configured for transporting the subject from a first position (128) to a second position (228), wherein in the second position the subject is at least partially within the imaging zone;
- a camera system (124) configured for acquiring optical image data (160) descriptive of the support surface and the subject when subject support is in the first position;
- a user interface (136);
- a memory (138) storing machine executable instructions (150) and an artificial intelligence system (152), wherein the artificial intelligence system is configured to output scan label data (162) in response to receiving the optical image data as input;
- a computational system (130) configured for controlling the medical imaging system, wherein execution of the machine executable instructions causes the computational system to:
- control (300) the camera system to acquire the optical image data;
- receive (302) the scan label data by inputting the optical image data into the artificial intelligence system;
- render (304) the scan position data as an overlay (170, 500) using the user interface;
- provide (306) a scan label data modifier control (168) using the user interface, wherein the scan label data modifier control is configured to receive modified scan label data (170); and
- construct (308) annotated training data (172) from the optical image data and the modified scan label data if the scan label data is received.

2. The medical imaging system of claim 1, wherein execution of the machine executable instructions further causes the processor to:
- train the artificial intelligence system using the annotated training data immediately; and
- delete the annotated training data after training.

3. The medical imaging system of claim 1, wherein execution of the machine executable instructions further causes the processor to log the annotated training data.

4. The medical imaging system of claim 1, 2, or 3, wherein the artificial intelligence system comprises a convolutional neural network (154) and a scan position data generator (156), wherein the convolutional neural network is configured for outputting the scan label data in response to receiving the optical image data, and wherein the scan position data generator is configured for generating the scan position data in response to receiving the scan label data.

5. The medical imaging system of claim 4, wherein the scan position data generator comprises any one of the following: a rule-based system, a trained neural network, a decision tree, a support vector machine, a principal component analysis algorithm, and combinations thereof.

6. The medical imaging system of any one of the preceding claims, wherein the user interface comprises a projection system configured for projecting the overlay on the subj ect.

7. The medical imaging system of any one of the preceding claims, wherein the user interface comprises a screen configured for displaying the optical image data and the overlay on the optical image data.

8. The medical imaging system of any one of the preceding claims, wherein the scan label data modifier control is implemented using any one of the following: a gesture recognition system, a touch screen, a mouse, a graphical user interface, and combinations thereof.

9. The medical imaging system of any one of the preceding claims, wherein the scan label data comprise any one of the following: a subject pose, a subject position, subject outline, a subject weight estimate, a subject skeletal overlay, an organ identification, a location of a region of interest, a cable, an antenna location, a support location, a subject outline, a tube location, a contrast agent injector location, a medical equipment location, and combinations thereof.

10. The medical imaging system of any one of the preceding claims, wherein execution of the machine executable instructions further causes the processor to receive scan parameters, wherein the artificial intelligence system is configured to output the scan label data in response to receiving the optical image data and the scan parameters as input.

11. The medical imaging system of claim 10, wherein the scan parameters comprise any one of the following: an operator identifier, an ordering physician identifier, a location identifier, a medical imaging protocol identifier, a magnetic resonance imaging antenna identifier, and combinations thereof.

12. The medical imaging system of any one of the preceding claims, wherein the medical imaging system is any one of the following: a magnetic resonance imaging system, a computed tomography system, a positron emission tomography system, and a single photon emission tomography system.

13. The medical imaging system of any one of the preceding claims, wherein the memory contains medical imaging system control commands configured to control the medical imaging system to acquire the tomographic medical image data according to a medical imaging protocol, wherein execution of the machine executable instructions further causes the processor to:
- modify the medical imaging control commands using the scan label data; and
- control the magnetic resonance imaging system with medical imaging system control commands to acquire the tomographic medical image data.

14. A method of operating a medical imaging system (100) configured for acquiring tomographic medical imaging data (256) from a subject (118) within an imaging zone (108), wherein the medical imaging system comprises a subject support (120) with a support surface (122), wherein the support surface is configured to receive and support a subject, wherein the subject support is configured for transporting the subject from a first position (128) to a second position (228), wherein in the second position the subject is at least partially within the imaging zone, wherein the medical imaging system further comprises a camera system (124) configured for acquiring optical image data (160) descriptive of the support surface and the subject when subject support is in the first position, wherein the medical imaging system further comprises a user interface (136), wherein the medical imaging system further comprises a memory (138) storing an artificial intelligence system (152), wherein the artificial intelligence system is configured to output scan label data (162) in response to receiving the optical image data as input, wherein the method comprises:
- controlling (300) the camera system to acquire the optical image data;
- receiving (302) the scan label data by inputting the optical image data into the artificial intelligence system;
- rendering (304) the scan position data as an overlay (166, 500) using the user interface;
- providing (306) a scan label data modifier control (168) using the user interface, wherein the scan label data modifier control is configured to receive modified scan label data; and
- constructing (308) annotated training data (172) from the optical image data and the modified scan label data if the scan label data is received.

15. A computer program comprising machine executable instructions (150) for execution by a computational system (130) controlling a medical imaging system (100), wherein the medical imaging system is configured for acquiring tomographic medical imaging data (256) from a subject (118) within an imaging zone (108), wherein the medical imaging system comprises:
- a subject support (120) with a support surface (122), wherein the support surface is configured to receive and support a subject (118), wherein the subject support is configured for transporting the subject from a first position (128) to a second position (228), wherein in the second position the subject is at least partially within the imaging zone;
- a camera system (124) configured for acquiring optical image data (160) descriptive of the support surface and the subject when subject support is in the first position;
- a user interface (136);
- a memory (138) storing machine executable instructions and an artificial intelligence system (152), wherein the artificial intelligence system is configured to output scan label data (162) in response to receiving the optical image data as input;
wherein execution of the machine executable instructions causes the computational system to:
- control (300) the camera system to acquire the optical image data;
- receive (302) the scan label data by inputting the optical image data into the artificial intelligence system;
- render (304) the scan position data as an overlay (166, 500) using the user interface;
- provide (306) a scan label data modifier control (168) using the user interface, wherein the scan label data modifier control is configured to receive modified scan label data; and
- construct (308) annotated training data (172) from the optical image data and the modified scan label data if the scan label data is received.
